# EUROPEAN PATENT APPLICATION

(11) **EP 4 656 733 A1**
(43) Date of publication of application: **03.12.2025**
(21) Application number: 25179681.9
(22) Date of filing: 29.05.2025
(51) Int. Cl.: C12Q 1/00, G01N 27/327

(54) **ELECTROCHEMICAL BIOSENSOR, PREPARATION METHOD, AND USE**

(30) Priority: 31.05.2024 CN 202410705555
(71) Applicant: Shanghai United Imaging Microelectronics Technology Co., Ltd., Shanghai 201800 (CN)
(72) Inventor: DING, Peng, Shanghai 201800 (CN)
(74) Representative: Murgitroyd & Company

(57) **Abstract**

An electrochemical biosensor including: an electrode; an electron mediator; and a biological enzyme; the electron mediator and the biological enzyme are disposed on a surface of the electrode, wherein the electron mediator comprises a transition metal complex directly bonded to the electrode via a chemical bond, and the biological enzyme is directly bonded to the electrode via a chemical bond or the biological enzyme is physically adsorbed onto the electrode.

## Description

### CROSS-REFERENCE TO RELATED APPLICATION

This application claims priority to Chinese Patent Application No. 202410705555.X, filed on May 31, 2024.

### TECHNICAL FIELD

The present application relates to sensors, and in particular, to a electrochemical biosensor and preparation method and use.

### BACKGROUND

Electrochemical biosensors utilize immobilized biological enzymes to selectively recognize analytes and establish a correlation between electrical signals and analyte concentrations. In such biosensors, the efficient transfer of electrons generated during the enzymatic reaction between the biological enzyme and the analyte to the electrode is critical for rapid and accurate analyte detection. However, due to structural limitations of biological enzymes (e.g., glucose oxidase), such as steric hindrance or insufficient electrical conductivity at the active site, direct electron transfer to the electrode is often inefficient. Consequently, electron mediators are typically required to facilitate this electron transfer.

Most existing techniques involve immobilizing biological enzymes and polymer-grafted electron mediators through cross-linking reactions, encapsulating the enzymes within a polymer network and depositing the composite onto the electrode surface. This approach necessitates excessive loading of biological enzymes and electron mediators, leading to suboptimal efficiency of electron mediation. Additionally, the immobilized electron mediators and enzymes may detach from the electrode over time during use, compromising the stability and sensitivity of the electrochemical biosensor and potentially rendering it inoperative. Furthermore, leachable electron mediators (e.g., transition metal complexes) may migrate into biological systems, posing significant cytotoxicity risks and safety concerns for in vivo applications.

Therefore, it is desirable to provide an electrochemical biosensor and preparation method and use.

### SUMMARY

An aspect of the present application may provide an electrochemical biosensor comprising: an electrode; an electron mediator; and a biological enzyme; the electron mediator and the biological enzyme are disposed on a surface of the electrode, wherein the electron mediator comprises a transition metal complex directly bonded to the electrode via a chemical bond, and the biological enzyme is directly bonded to the electrode via a chemical bond or the biological enzyme is physically adsorbed onto the electrode.

In some embodiments, the transition metal complex, biological enzyme, and electrode are connected in a configuration selected from the group consisting of:
(a) a first reactive group of the transition metal complex forms a chemical bond with a second reactive group on a surface of the electrode, and the biological enzyme forms a chemical bond with the second reactive group on a surface of the electrode or the biological enzyme is physically adsorbed onto a surface of the electrode;
(b) a first reactive group of the transition metal complex forms a chemical bond with a third reactive group on a branched molecular chain attached to a surface of the electrode, and the biological enzyme is physically adsorbed onto a surface of the electrode;
(c) a first reactive group of the transition metal complex forms a chemical bond with a third reactive group on a branched molecular chain attached to a surface of the electrode, and the biological enzyme forms a chemical bond with a second reactive group on a surface of the electrode.

In some embodiments, the transition metal complex is selected from at least one of ruthenium complexes, or osmium complexes, and the first reactive group is selected from at least one of an amino group, an aldehyde group, an epoxy group, or a carboxyl group.

In some embodiments, the second reactive group is selected from at least one of a hydroxyl group, an amino group, or a carboxyl group.

In some embodiments, the branched molecular chain comprises a polymer selected from at least one of polyepoxide, polyalkane, polyether, polyester, polyamide, polyamide ester, polyurethane, polysiloxane, or polycarbosilane, and the third reactive group is selected from at least one of a hydroxyl group, an amino group, an epoxy group, or a carboxyl group.

In some embodiments, the branched molecular chain has a relative molecular weight of 100 to 5,000.

In some embodiments, the biological enzyme is selected from at least one of glucose oxidase, ethanol oxidase, lactate dehydrogenase, uric acid oxidase, acetylcholinesterase, or horseradish peroxidase.

In some embodiments, the electrochemical biosensor further comprising a functional film layer disposed on the electrode.

Another aspect of the present application may provide a method for preparing an electrochemical biosensor, comprising: step (a): activating an electrode comprising reactive groups, and chemically bonding a transition metal complex to the activated electrode to form a transition metal complex-modified electrode; step (b): immobilizing a biological enzyme onto the transition metal complex-modified electrode to form the electrochemical biosensor.

In some embodiments, the transition metal complex-modified electrode in step (a) satisfies at least one of the following conditions:
(i) the reactive groups on the electrode include a second reactive group and/or a branched relative molecular chain comprising a third reactive group;
(ii) the activating an electrode comprising reactive groups comprises contacting the electrode with an activator solution selected from at least one of: a mixture of dicyclohexylcarbodiimide (DCC) and 4-dimethylaminopyridine (DMAP); a mixture of diisopropylcarbodiimide (DIC) and DMAP; a mixture of DCC and 4-pyrrolidinopyridine; a mixture of DIC and 4-pyrrolidinopyridine; a mixture of 1-ethyl-3-(3-dimethylaminopropyl)carbodiimide (EDC) and N-hydroxysuccinimide (NHS); an acyl chloride solution; or an anhydride solution; wherein the activation is performed at 0°C to 100°C for 0.5 hour to 72 hours;
(iii) in chemically bonding a transition metal complex to the activated electrode, placing the electrode in a transition metal complex solution for reaction under conditions comprising: reaction temperature: 0°C to 100°C; reaction duration: 1 hour to 72 hours; mass concentration of said transition metal complex solution: 0.01 wt% to 10 wt%; wherein said transition metal complex solution is selected from the group consisting of ruthenium complex solutions, osmium complex solutions, and combinations thereof.

In some embodiments, the biological enzyme is immobilized by: (i) reacting the transition metal complex-modified electrode with a biological enzyme solution in the presence of an activator solution; or (ii) contacting the transition metal complex-modified electrode with a biological enzyme solution and a crosslinking agent.

In some embodiments, the biological enzyme solution has a mass concentration of 0.01% to 10% and is selected from at least one of glucose oxidase, ethanol oxidase, lactate dehydrogenase, uric acid oxidase, acetylcholinesterase, or horseradish peroxidase; the crosslinking agent is selected from at least one of glutaraldehyde, polyethylene glycol diglycidyl ether, dicarboxylic acid, dimethyl adiponimide, dimethyl adipic acid, dimethyl pyrimidate, dimethyl malonate, 3,3'-dithiobispropionimidate dimethyl ester dihydrochloride, 3,3' -dithiobis(sulfosuccinimidyl propionate), ethylene glycol bis(sulfosuccinimidyl succinate), carbodiimide hydrochloride, or N-cyclohexyl-N' -(2-morpholinoethyl)carbodiimide methyl p-toluenesulfonate.

Another aspect of the present application may provide use of the electrochemical biosensor as mentioned before for detecting glucose, lactic acid, ethanol, uric acid, or acetylcholine ester or horseradish peroxide.

Additional features will be set forth in part in the description which follows, and in part will become apparent to those skilled in the art upon examination of the following and the accompanying drawings or may be learned by production or operation of the examples. The features of the present application may be realized and attained by practice or use of various aspects of the methodologies, instrumentalities, and combinations set forth in the detailed examples discussed below.

### BRIEF DESCRIPTION OF THE DRAWINGS

The present application is further illustrated in terms of exemplary embodiments. These exemplary embodiments are described in detail with reference to according to the drawings. These embodiments are non-limiting exemplary embodiments, in which like reference numerals represent similar structures, and wherein:
FIG. 1 is a schematic diagram of the electron mediator and biological enzyme bonded to the electrode in the electrochemical biosensor of Example 3 of the present invention, where A represents the biological enzyme and B represents the electron mediator;
FIG. 2 shows cyclic voltammetry curves of the electrochemical biosensor prepared in Example 1 of the present invention and Proportional Example 1, where a corresponds to Example 1 and b corresponds to Proportional Example 1;
FIG. 3 illustrates the relationship between current and time for the electrochemical biosensors prepared in Example 1 and Proportional Example 1 when exposed to the same concentration of glucose solution, where c represents Example 1 and d represents Proportional Example 1;
FIG. 4 depicts the linear relationship between response current and glucose concentration for the electrochemical biosensors prepared in Examples 1-3 and Proportional Examples 1-3 of the present invention, where e corresponds to Example 1, f to Example 2, g to Example 3, h to Proportional Example 1, i to Proportional Example 2, and j to Proportional Example 3.

### DETAILED DESCRIPTION

In the following detailed description, numerous specific details are set forth by way of examples in order to provide a thorough understanding of the relevant disclosure. However, it should be apparent to those skilled in the art that the present application may be practiced without such details. In other instances, well-known methods, procedures, systems, components, and/or circuitry have been described at a relatively high level, without detail, in order to avoid unnecessarily obscuring aspects of the present application. Various modifications to the disclosed embodiments will be readily apparent to those skilled in the art, and the general principles defined herein may be applied to other embodiments and applications without departing from the spirit and scope of the present application. Thus, the present application is not limited to the embodiments shown, but is to be accorded the widest scope consistent with the claims.

The terminology used herein is for the purpose of describing particular example embodiments only and is not intended to be limiting. As used herein, the singular forms "a", "an", and "the" may be intended to include the plural forms as well, unless the context clearly indicates otherwise. It will be further understood that the terms "comprise", "comprises", and/or "comprising", "include", "includes", and/or "including", when used in this specification, specify the presence of stated features, integers, steps, operations, elements, and/or components, but do not preclude the presence or addition of one or more other features, integers, steps, operations, elements, components, and/or groups thereof.

It will be understood that the terms "system", "engine", "unit", "module", and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on", "connected to", or "coupled to", another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The terms "pixel" and "voxel" in the present application are used interchangeably to refer to an element of an image.

These and other features, and characteristics of the present application, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present application. It is understood that the drawings are not to scale.

Most existing electrochemical biosensor technologies involve blending a biological enzyme with a polymer grafted with an electron mediator, encapsulating the enzyme within a polymer network via crosslinking reactions, and depositing the composite onto an electrode surface. However, this immobilization strategy renders the electron transfer efficiency between the enzyme and the mediator contingent upon their relative ratios. To achieve optimal electron transfer, excessive loadings of both components are required, leading to suboptimal utilization of the electron mediator. Additionally, the adsorbed enzyme and mediator may detach from the electrode during prolonged use, compromising the biosensor's stability, sensitivity, and operational longevity. Detached mediators, particularly transition metal complexes, may leach into biological environments, posing cytotoxicity risks and safety concerns.

Accordingly, there exists a need for an improved electrochemical biosensor, its preparation method, and applications thereof. The disclosed biosensor addresses these challenges by chemically bonding the electron mediator directly to the electrode surface, enabling efficient direct electron transfer and robust attachment. This configuration enhances electron transfer efficiency, maximizes mediator utilization, and improves the biosensor's stability, sensitivity, and biocompatibility.

The present application employs a transition metal complex covalently bonded to the electrode, which not only facilitates direct electron transfer between the mediator and the electrode-thereby improving electron transfer efficiency and mediator utilization-but also strengthens the mediator-electrode interface. This covalent attachment mitigates mediator leakage, reduces potential biological toxicity, and enhances the biosensor's operational stability, sensitivity, and safety.

Furthermore, when the biological enzyme is also covalently bonded to the electrode, the enzyme's structural stability is enhanced, promoting efficient electron transfer between the enzyme and the electrode. This dual covalent bonding strategy further improves the overall performance of the electrochemical biosensor.

In summary, the disclosed electrochemical biosensor leverages chemical bonding between the electron mediator and the electrode to achieve direct electron transfer, robust attachment, and improved mediator utilization. These features collectively enhance the biosensor's stability, sensitivity, and biosafety, addressing critical limitations of prior art devices.

The electrochemical biosensor of the present invention comprises an electrode, an electron mediator, and a biological enzyme disposed thereon, wherein the electron mediator is a transition metal complex covalently bonded to the electrode, and the biological enzyme is either covalently bonded to the electrode or physically adsorbed thereon.

Optionally, the connection configurations between the transition metal complex, the biological enzyme, and the electrode comprise:
(1) A first reactive group of the transition metal complex forms a chemical bond with a second reactive group on the electrode surface, while the biological enzyme either forms a chemical bond with the second reactive group on the electrode or is physically adsorbed thereon;
(2) The first reactive group of the transition metal complex forms a chemical bond with a third reactive group of a branched molecular chain attached to the electrode, and the biological enzyme is physically adsorbed onto the electrode;
(3) The first reactive group of the transition metal complex forms a chemical bond with a third reactive group of a branched molecular chain attached to the electrode, and the biological enzyme forms a chemical bond with the second reactive group on the electrode.

In configurations (1) to (3), while configuration (1) enables direct covalent bonding of both the electron mediator and the biological enzyme to the electrode, the branched molecular chain in configurations (2) and (3) offers distinct advantages. The branched structure provides multiple reactive sites (i.e., a higher density of third reactive groups) per unit molar amount compared to the second reactive groups on the electrode surface. This allows for the attachment of a greater number of electron mediators per branched chain, with the mediators radially distributed around the electrode surface. Such spatial arrangement increases the probability of interaction between the electron mediators and the biological enzyme, thereby enhancing electron mediator utilization efficiency.

Furthermore, the branched molecular chains introduce steric hindrance, which promotes an interlaced distribution of electron mediators and biological enzymes on the electrode surface. This structured arrangement facilitates efficient electron transfer between the biological enzyme and the electron mediator by minimizing diffusion distances and optimizing spatial proximity.

Accordingly, when the biological enzyme is physically adsorbed (i.e., non-covalently attached) to the electrode, configuration (2) is preferred to achieve covalent bonding of the electron mediator to the electrode. Conversely, when the biological enzyme is also covalently attached to the electrode, configuration (3) is preferred to establish a covalent connection between the electrode and the electron mediator, and establish a covalent connection between the electrode and the biological enzyme.

It should be noted that by designing differential reactivity between the second reactive group on the electrode surface and the third reactive group of the branched molecular chain toward the first reactive group of the electron mediator (e.g., selecting the first reactive group as an amino group, the second reactive group as a hydroxyl group, and the third reactive group as an epoxy group, or selecting the first reactive group as an amino group, the second reactive group as an amino group, and the third reactive group as an epoxy group), the reactivity between the first and third reactive groups is designed to exceed that between the first and second reactive groups. This design ensures that the first reactive group of the transition metal complex preferentially reacts with the third reactive group of the branched molecular chain, such that when both the branched molecular chain and second reactive groups are present on the electrode, the transition metal complex selectively forms covalent bonds exclusively with the branched molecular chain.

The electrochemical biosensor of the present invention leverages the selective chemical reaction and covalent bond formation between the first reactive group of the transition metal complex and the second reactive group on the electrode surface; and/or covalent bond formation between the first reactive group of the transition metal complex and the branched molecular chain on the electrode surface. This strategy enables direct covalent attachment of the electron mediator to the electrode, facilitating efficient direct electron transfer between the electron mediator and the electrode. The covalent bonding not only enhances electron transfer efficiency and electron mediator utilization but also strengthens the mediator-electrode interface, effectively mitigating mediator leakage and reducing potential biological toxicity. These improvements collectively enhance the biosensor's stability, sensitivity, and biocompatibility.

Additionally, when the electrode surface bears second reactive groups, these groups can form covalent bonds with the biological enzyme, immobilizing the enzyme onto the electrode. This covalent immobilization further improves the electrode's structural stability and promotes efficient electron transfer between the biological enzyme and the electrode, thereby enhancing the overall performance of the electrochemical biosensor.

**In** summary, the disclosed electrochemical biosensor achieves direct electron transfer and robust bonding between the electron mediator and the electrode through selective covalent bonding strategies. These design features effectively improve electron transfer efficiency, maximize electron mediator utilization, and enhance the biosensor's stability, sensitivity, and biosafety.

Optionally, the transition metal complex is selected from ruthenium complexes and/or osmium complexes, and the first reactive group is selected from at least one of an amino group, an aldehyde group, an epoxy group, or a carboxyl group; the second reactive group is selected from at least one of a hydroxyl group, an amino group, or a carboxyl group; the branched molecular chain is selected from at least one of polyepoxide, polyalkane, polyether, polyester, polyamide, polyamide ester, polyurethane, polysiloxane, and polycarbosilane, and the third reactive group is selected from at least one of a hydroxyl group, an amino group, an epoxy group, or a carboxyl group. This configuration enables efficient covalent bonding between the electron mediator and the electrode ; and/or the configuration enables efficient covalent bonding between biological enzyme and the electrode, thereby enhancing the electrochemical sensor's electron transfer efficiency and overall performance.

It should be noted that in practical applications, the choice of the second and third reactive groups on the branched molecular chain can be designed to the nature of the first reactive group in the transition metal complex. For example, when the first reactive group in the transition metal complex is an amino group or a hydroxyl group, the third reactive group can be an epoxy group, enabling ring-opening reactions to form covalent bonds. Conversely, when the first reactive group is an epoxy group, the third reactive group can be an amino group or a hydroxyl group. This designed approach optimizes covalent bonding between the electron mediator and the electrode; and/or the designed approach optimizes covalent bonding between the biological enzyme and the electrode.

Optionally, the branched molecular chain has a relative molecular mass in the range of 100 to 5000, preferably 100 to 1000. Controlling the molecular weight of the branched molecular chain enables precise regulation of the distance between the electron mediator attached to the chain and the biological enzyme. This spatial optimization facilitates efficient electron transfer from the analyte - analyte oxidase reaction to the electron mediator and subsequently to the electrode, further enhancing electron transfer efficiency.

Optionally, the biological enzyme is selected from at least one of glucose oxidase, ethanol oxidase, lactate dehydrogenase, uric acid oxidase, acetylcholinesterase, or horseradish peroxidase.

Optionally, the electrochemical biosensor further comprises a functional membrane layer formed on the electrode.

**In** some embodiments, the functional membrane layer can be classified based on its function into an analyte flux - limiting film layer, an interference - limiting film layer, and a biocompatible film layer.

**In** one embodiment, the analyte - flux limiting film layer, interference - limiting film layer, and biocompatible film layer are independently selected from at least one of the following: polyurethane film layer, poly(4 - vinylpyridine - pyridine - co - styrene) film layer, poly(4 - vinylpyridine - pyridine - co - styrene) derivative film layer, poly(2 - vinylpyridine - pyridine - co - styrene) film layer, poly(2 - vinylpyridine - pyridine - co - styrene) derivative film layer, poly(4 - vinylpyridine) film layer, poly(4 - vinylpyridine) derivative film layer, poly(2 - vinylpyridine) film layer, poly(2 - vinylpyridine) derivative film layer, poly(4 - vinylpyridine co - butyl methacrylate) film layer, poly(4 - vinylpyridine co - butyl methacrylate) derivative film layer, poly(2 - vinylpyridine co - butyl methacrylate) film layer, poly(2 - vinylpyridine co - butyl methacrylate) derivative film layer, polyvinylpyrrolidone film layer, polylysine film layer, perfluorosulfonic acid (Nafion) film layer, polyethylene glycol film layer, sodium alginate film layer, or polysaccharide film layer.

Meanwhile, the present application also provides a method for preparing an electrochemical biosensor, comprising the following steps:
S1, activating an electrode having reactive groups, and then directly connecting a transition metal complex to the electrode via a chemical bond to obtain an electrode bonded with a transition metal complex;
S2, immobilizing a biological enzyme on the electrode bonded with a transition metal complex to obtain an electrochemical biosensor.

In one embodiment, in step S1, the reactive group comprises a second reactive group and/or a molecular chain with a branched structure, wherein the branched structure comprises a third reactive group.

When preparing an electrode with a second reactive group, the surface of the electrode can be treated by methods such as plasma treatment or chemical vapor deposition.

When preparing electrodes with molecular chains which having branched structures, the following two implementations can be adopted:
(I) The first implementation: Placing the electrode directly into a solution of a compound having a branched structure to react and form the molecular chain with a branched structure, thereby obtaining an electrode with the molecular chain having a branched structure on its surface.
(II) The second implementation: treating the electrode surface by methods such as plasma treatment or chemical vapor deposition to obtain an electrode with a second reactive group on its surface, and then placing the electrode with a second reactive group on its surface into a solution of a compound with a branched structure for reaction to form a molecular chain with a branched structure on the surface, thereby obtaining an electrode with a molecular chain having a branched structure on its surface. Compared with the first implementation, this implementation can improve the grafting rate and uniformity of the molecular chains with branched structures on the electrode surface. Therefore, the second implementation is preferred in some embodiments.

In addition, in one embodiment, referring to the second implementation, by controlling the concentration of the solution of a compound with a branched structure and adjusting the grafting rate of the molecular chain with a branched structure on the surface, an electrode having both the molecular chain with a branched structure and the second reactive group can be obtained.

In one embodiment, the solution of a compound with a branched structure is selected from at least one of the following: branched polyalkylene oxide s solution, branched polyalkane solution, branched polyether solution, branched polyester solution, branched polyamide solution, branched polyamide ester solution, branched polyurethane solution, branched polysiloxane solution, or branched polycarbosilane solution.

Optionally, in step S1, the activating an electrode having reactive groups includes activating the electrode with reactive groups using an activator solution. The activator solutions are selected from at least one of the following: a mixture of dicyclohexylcarbodiimide (DCC) and 4-dimethylaminopyridine (DMAP), a mixture of diisopropylcarbodiimide (DIC) and DMAP, a mixture of DCC and 4-pyrrolidinopyridine, a mixture of DIC and 4-pyrrolidinopyridine, a mixture of 1-ethyl-3-(3-dimethylaminopropyl)carbodiimide (EDC) and N-hydroxysuccinimide (NHS), an acyl chloride solution, or an anhydride solution. The activation is performed at a temperature of 0°C to 100°C for 0.5 hours to 72 hours. This configuration activates the second and/or third reactive groups on the electrode surface or within the branched molecular chains, facilitating subsequent reactions between the branched molecular chains and/or second reactive groups with the first reactive group of the transition metal complex, thereby enabling direct covalent attachment of the electron mediator to the electrode.

Optionally, the electrode is placed in a transition metal complex solution for reaction under the following conditions: reaction temperature of 0°C to 100°C, reaction time of 1 hour to 72 hours, and mass concentration of the transition metal complex solution of 0.01% to 10%. The transition metal complex solution is selected from ruthenium complex solutions and/or osmium complex solutions.

In one embodiment, prior to step S2, the electrode bonded with a transition metal complex is washed with a solvent such as water, ethanol, or phosphate-buffered saline (PBS buffer) to remove unbound transition metal complex.

In step S2, methods for immobilizing a biological enzyme on the electrode bonded with a transition metal complex comprise:
Method 1: Reacting the biological enzyme solution with the electrode bonded with a transition metal complex in an activator solution to achieve direct covalent bonding of the biological enzyme to the electrode. The activator solution is selected from at least one of the following: a DCC/DMAP mixture, a DIC/DMAP mixture, a DCC/4-pyrrolidinopyridine mixture, a DIC/4-pyrrolidinopyridine mixture, an EDC/NHS mixture, an acyl chloride solution, or an anhydride solution. The reaction is conducted at 0°C to 100°C for 0.5 hours to 72 hours.
Method 2: Contacting the biological enzyme solution and a crosslinking agent with the electrode bonded with a transition metal complex surface, whereby the biological enzyme is immobilized through a crosslinking reaction mediated by the crosslinking agent (i.e., physical adsorption). The crosslinking agent is selected from dialdehyde solution. Specifically, the dialdehyde solution is selected from at least one of : glutaraldehyde; dicarboxylic acid; dimethyl adiponimide; dimethyl adipate; dimethyl pyrimidate; dimethyl malonate; 3,3' -dithiodipropionic acid dihydrochloride; 3,3' -dithiobis(sulfosuccinimidyl propionate); ethylene glycol bis(sulfosuccinimidyl succinate); carbodiimide hydrochloride; or N-cyclohexyl-N' -(2-morpholinoethyl)carbodiimide methyl-p-toluenesulfonate.

It is understood that method 1 is selected when the biological enzyme is to be covalently bonded to the electrode, while method 2 is used for physical adsorption of the biological enzyme.

Optionally, the mass concentration of the biological enzyme solution is 0.01% - 10%, preferably 0.1% - 3%.

Optionally, the biological enzyme solution is selected from at least one of a glucose oxidase solution, an ethanol oxidase solution, a lactate dehydrogenase solution, an uric acid oxidase solution, an acetylcholinesterase solution, or a horseradish peroxidase solution.

Optionally, after the biological enzyme is immobilized on the transition metal complex - bonded electrode, it further comprises forming a functional layer on the transition metal complex - bonded electrode.

**In** one embodiment, a method for forming the functional layer on the transition metal complex - bonded electrode includes: placing a functional film solution on the transition metal complex - bonded electrode and curing it to form a functional film layer; wherein the functional film solution comprises at least one of a first polymer solution, a second polymer solution, or a third polymer solution.

It can be understood that when the functional membrane solution includes a first polymer solution, a second polymer solution, and/or a third polymer solution, the first polymer solution, the second polymer solution, and/or the third polymer solution are placed successively on the transition metal complex - bonded electrodes when preparing the functional membrane layer.

Further, the first polymer solution, the second polymer solution, and the third polymer solution are independently selected from at least one of the following:
polyurethane; poly(4 - vinylpyridine - co - styrene); poly(4 - vinylpyridine - co - styrene) derivative; poly(2 - vinylpyridine - co - styrene); poly(2 - vinylpyridine - co - styrene) derivative; poly(4 - vinylpyridine); poly(4 - vinylpyridine) derivatives; poly(2 - vinylpyridine); poly(2 - vinylpyridine) derivatives; poly(4 - vinylpyridine co - butyl methacrylate); poly(4 - vinylpyridine co - butyl methacrylate) derivatives; poly(2 - vinylpyridine co - butyl methacrylate); poly(2 - vinylpyridine co - butyl methacrylate) derivatives; polyvinylpyrrolidone; polylysine; perfluorosulfonic acid (Nafion); polyethylene glycol; sodium alginate; or polysaccharide.

Furthermore, the present invention also provides an application of an electrochemical biosensor as described above for the detection of glucose, lactic acid, ethanol, uric acid, acetylcholine ester or horseradish peroxide.

In one embodiment, the electrochemical biosensor is applied as a working electrode in a glucose sensor. When implanted under the tester's skin, the glucose in the subcutaneous intercellular space diffuses through the functional membrane to the surface of the working electrode. The glucose reacts with the corresponding enzyme on the surface of the working electrode, generating electrical signals that are correlated with the concentration of glucose in the interstitial fluid, thereby reflecting human blood glucose levels.

The electrochemical biosensor, as well as its preparation method and application, will be further described through the following specific embodiments. However, those skilled in the art will understand that the following embodiments are for illustrative purposes only and should not be regarded as limiting the scope of the invention. Where no specific conditions are specified in the embodiments, the general conditions or the conditions recommended by the manufacturer shall apply. Reagents or instruments used, where the manufacturer is not specified, are regular products available through market purchase.

### Example 1

The electrode surface was treated by oxygen plasma to introduce carboxyl groups, and obtain a carboxyl-functionalized electrode. The carboxyl-functionalized electrode was then immersed in a dicyclohexylcarbodiimide (DCC)/4-dimethylaminopyridine (DMAP) mixed solution for activation at 25°C for 12 hours, yielding an activated electrode. The activated electrode was immersed in a 0.2% (w/v) solution of 3-(glycidyloxy)-2,2-bis[(glycidyloxy)methyl]propanol (relative molecular weight 304) at 25°C for 24 hours to graft branched molecular chains bearing epoxy groups onto the surface, and obtain a branched-chain-functionalized electrode.

The branched-chain-functionalized electrode was then incubated in a 0.3% (w/v) solution of (1,1'-dimethyl-1H,1'H-[2,2']biimidazol)-6-(1H-methyl-1,1"H-[2,2'-biimidazol]-1-yl)-1-amine osmium complex at 25°C for 24 hours. The electrode was washed with water to remove unbound osmium complex, resulting in a clean electrode with covalently bonded osmium mediator.

A 0.4% glucose oxidase solution and glutaraldehyde were applied to the surface of the electrode with covalently bonded osmium mediator, followed by successive coatings of Nafion and sulfonate-modified poly(4-vinylpyridine-co-styrene) sequentially. After curing at 37°C for 72 hours, a functional film layer was formed, yielding the electrochemical biosensor.

### Example 2

The procedure was identical to Example 1, except that the carboxyl-functionalized electrode prepared in Example 1 was immersed in a mixed solution of 1-ethyl-3-(3-dimethylaminopropyl)carbodiimide (EDC) and N-hydroxysuccinimide (NHS) for activation. The activation was performed at 30°C for 18 hours to obtain an activated electrode. The activated electrode was then immersed in a 0.1% (w/v) solution of (1,1'-dimethyl-1H,1'H-[2,2']biimidazol)-6-(1H-methyl-1,1"H-[2,2'-biimidazol]-1-yl)-1-amine osmium complex at 20°C for 18 hours to bond the osmium complex to the surface. A final electrochemical biosensor was prepared under otherwise identical conditions.

### Example 3

The only difference from Example 1 is that the activated electrode was immersed in a 0.3% (w/v) trimethylpropane diglycidyl ether solution and reacted at 30°C for 18 hours, yielding an electrode with branched molecular chains and carboxyl groups on the surface. The electrode with branched molecular chains and carboxyl groups was then immersed in a 1% (w/v) solution of (1,1'-dimethyl-1H,1'H-[2,2']biimidazol)-6-(1H-methyl-1,1"H-[2,2'-biimidazol]-1-yl)-1-amine osmium complex at 25°C for 24 hours to bond the osmium complex and carboxyl groups to the surface. The electrode was washed with water to remove unbound osmium complex, resulting in a clean electrode with covalently bonded osmium mediator and carboxyl groups.

A 0.4% glucose oxidase solution and the electrode with covalently bonded osmium mediator and carboxyl groups were added to a mixed solution of 1-ethyl-3-(3-dimethylaminopropyl)carbodiimide (EDC) and N-hydroxysuccinimide (NHS), and reacted at 25°C for 24 hours to covalently immobilize glucose oxidase on the electrode. An electrochemical biosensor was prepared under otherwise identical conditions.

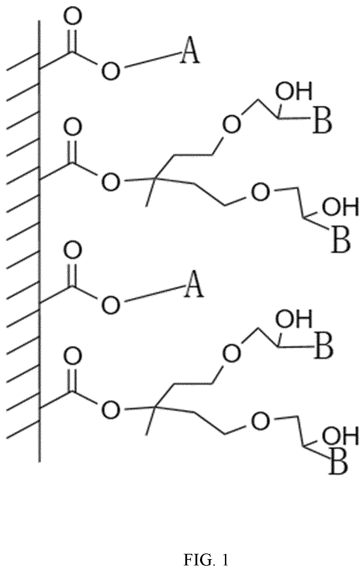

FIG. 1 is a schematic diagram of the electron mediator and biological enzyme bonded to the electrode surface in the electrochemical biosensor of this embodiment. As shown in FIG. 1, electron mediators and biological enzymes are alternately distributed on the electrode surface via covalent bonds.

### Example 4

The only difference from Example 2 is that the mass concentration of the (1,1'-dimethyl-1H,1'H-[2,2']biimidazol)-6-(1H-methyl-1,1"H-[2,2'-biimidazol]-1-yl)-1-amine osmium complex solution was 0.05% (w/v). A 0.1% (w/v) glucose oxidase solution and the electrode with covalently bonded osmium mediator and carboxyl group were added to a mixed solution of 1-ethyl-3-(3-dimethylaminopropyl)carbodiimide (EDC) and N-hydroxysuccinimide (NHS) to form the electrode with covalently bonded glucose oxidase and osmium complex. The electrochemical biosensor was obtained under otherwise identical conditions.

### Example 5

The only difference from Example 1 is that the electrode surface was treated by oxygen plasma to introduce hydroxyl groups, yielding a hydroxyl-functionalized electrode. The hydroxyl-functionalized electrode was immersed in an oxalyl chloride solution and reacted at 25°C for 2 hours. A 0.5% (w/v) solution of PAMAM dendrimer (N1,N24-bis(aminoethyl)-4,21-bis[3-[(2-aminoethyl)amino]propyl]-3-oxa-11,14-bis[3-[[2-[bis[3-[(2-aminoethyl)amino]-3-oxapropyl]amino]ethyl]amino]propyl]-8,17-dioxo-4,7,11,14,18,21-hexazatricosanediamide) was then applied, and the reaction was conducted at 25°C for 10 hours to graft branched molecular chains bearing amino groups onto the electrode surface, and obtain an amino-functionalized electrode. The amino-functionalized electrode was immersed in a 0.1% (w/v) solution of di(1,1'-dimethyl-1H,1'H-[2,2']biimidazol)(6-(1'-methyl-1H,1'H-[2,2'-biimidazol]-1-yl)hex-1-ene-epoxy) osmium complex under otherwise identical conditions to obtain an electrochemical biosensor.

### Example 6

The only difference from Example 1 is that a 0.1% (w/v) solution of second-generation hyperbranched bis-MPA polyester-16-hydroxyl was used to replace the 0.2% (w/v) 3-(glycidyloxy)-2,2-bis[(glycidyloxy)methyl]propanol. Additionally, a 0.3% (w/v) solution of (1,1'-dimethyl-1H,1'H-[2,2']biimidazol)(6-(1H-methyl-1,1"H-[2,2'-biimidazol]-1-yl)-1-carboxyl) osmium complex was used in place of the 0.3% (w/v) (1,1'-dimethyl-1H,1'H-[2,2']biimidazol)(6-(1H-methyl-1,1"H-[2,2'-biimidazol]-1-yl)-1-amine) osmium complex solution, with all other reaction conditions remaining identical. An electrochemical biosensor was obtained accordingly.

### Example 7

The only difference from Example 1 is that a 0.3% (w/v) ethanol oxidase solution was used in place of the 0.4% (w/v) glucose oxidase solution, carbodiimide hydrochloride was used instead of the glutaraldehyde solution, and a 0.3% (w/v) ruthenium complex solution bearing an amino group was used in place of the 0.3% (w/v) osmium complex solution bearing an amino group. All other reaction conditions remained identical, yielding an electrochemical biosensor.

### Proportional Example 1

The only difference from Example 1 is that an untreated electrode (i.e., lacking branched molecular chains on the surface) was dipped in a 0.3% (w/v) solution of (1,1'-dimethyl-1H,1'H-[2,2']biimidazol)(6-(1H-methyl-1,1"H-[2,2'-biimidazol]-1-yl)-1-amine) osmium complex. All other conditions remained identical, yielding an electrochemical biosensor.

### Proportional Example 2

The only difference from Proportional Example 1 is that a 0.3% (w/v) solution of tris(1,1'-dimethyl-1H,1'H-[2,2']biimidazol) osmium complex was used in place of the 0.3% (w/v) (1,1'-dimethyl-1H,1'H-[2,2']biimidazol)(6-(1H-methyl-1,1"H-[2,2'-biimidazol]-1-yl)-1-amine) osmium complex solution. All other conditions remained identical, yielding an electrochemical biosensor.

### Proportional Example 3

Comparative to Example 2, an electrochemical biosensor was prepared by activating a carboxyl-free electrode in a mixed solution of 1-ethyl-3-(3-dimethylaminopropyl)carbodiimide (EDC) and N-hydroxysuccinimide (NHS), with all other conditions being identical.

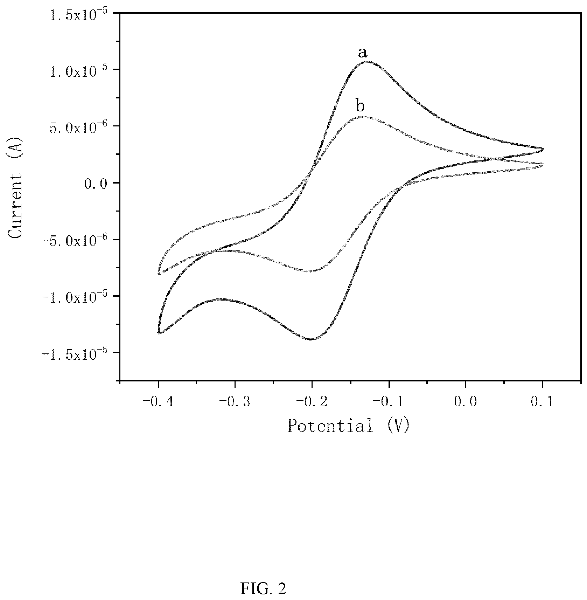

The electrochemical biosensors obtained from Example 1 and Proportional Example 1 were characterized by cyclic voltammetry. FIG. 2 displays the cyclic voltammetry curves of the electrochemical biosensors prepared in Example 1 and Proportional Example 1. The cyclic voltammetry procedure was as follows: The working electrode (WE), platinum counter electrode (CE), and silver/silver chloride reference electrode (RE) were connected to an electrochemical workstation and immersed in a 0.01 M PBS buffer at 37°C. The workstation was operated in cyclic voltammetry mode with a scanning potential range of -0.4 V to +0.1 V. As shown in FIG. 2, both electrodes exhibit typical redox peaks with close potentials, indicating successful covalent bonding of the electron mediator to the electrode surface.

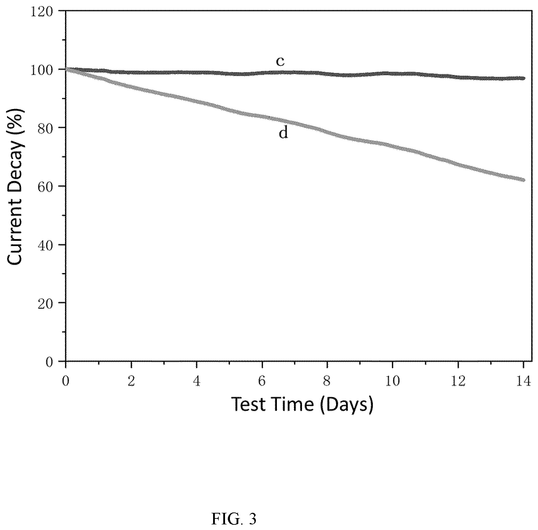

The stability of the electrochemical biosensors from Example 1 and Proportional Example 1 was evaluated, with results presented in FIG. 3. The testing method involved immersing the biosensors in a constant-concentration glucose solution (37°C) and measuring the generated current over 14 days. As evident from FIG. 3, the current in Example 1 showed minimal decay (approximately 3.2% over 14 days), whereas the current in Proportional Example 1 decayed significantly (38% over 14 days). These results demonstrate that the covalently bonded electron mediator maintains stability during use, mitigating current attenuation caused by mediator detachment inherent in traditional physical coating methods.

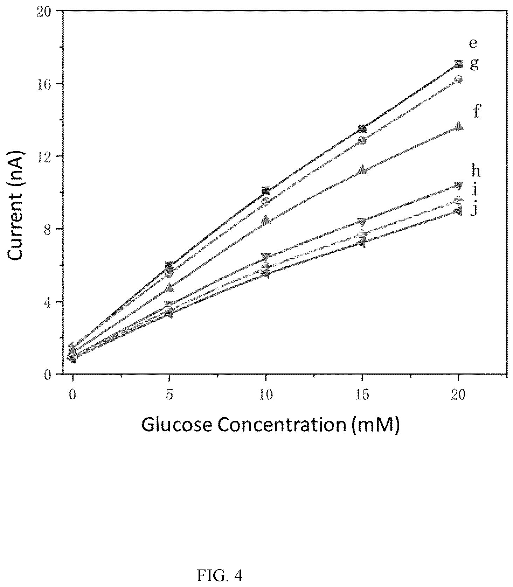

The sensitivities of the electrochemical biosensors prepared in Examples 1 to 3 and Proportional Examples 1 to 3 were tested, with results shown in Figure 4. The testing protocol entailed immersing the biosensors in glucose solutions of different concentrations and measuring the current response at each concentration.

As depicted in FIG. 4, Examples 1 to 3 exhibit higher sensitivity than Proportional Examples 1 to 3, demonstrating that covalent bonding of the electron mediator to the electrode surface enhances electron transfer efficiency, thereby improving the electrochemical sensor's sensitivity.

Collectively, the results in FIGS. 3 to 4 illustrate that the disclosed electrochemical biosensor, through covalent bonding between the electron mediator and the electrode, achieves efficient direct electron transfer and robust attachment. These features effectively enhance electron transfer efficiency, maximize mediator utilization, and improve the biosensor's stability, sensitivity, and biocompatibility.

The various technical features described in the embodiments may be combined in any manner. For brevity, not all possible combinations are explicitly recited herein; however, any non-contradictory combination of these features is intended to fall within the scope of this specification.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present application, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about", "approximate", or "substantially". For example, "about", "approximate", or "substantially" may indicate a certain variation (e.g., ±1%, ±5%, ±10%, or ±20%) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value".

## Claims

1. An electrochemical biosensor comprising:
an electrode; an electron mediator; and a biological enzyme; the electron mediator and the biological enzyme are disposed on a surface of the electrode,
wherein the electron mediator comprises a transition metal complex, and the transition metal complex directly bonded to the electrode via a chemical bond, and
the biological enzyme is directly bonded to the electrode via a chemical bond or the biological enzyme is physically adsorbed onto the electrode.

2. The electrochemical biosensor of claim 1, wherein the transition metal complex, biological enzyme, and electrode are connected in a configuration selected from the group consisting of:
(a) a first reactive group of the transition metal complex forms a chemical bond with a second reactive group on a surface of the electrode, and the biological enzyme forms a chemical bond with a second reactive group on a surface of the electrode or the biological enzyme is physically adsorbed onto a surface of the electrode;
(b) a first reactive group of the transition metal complex forms a chemical bond with a third reactive group on a branched molecular chain attached to a surface of the electrode, and the biological enzyme is physically adsorbed onto a surface of the electrode;
(c) a first reactive group of the transition metal complex forms a chemical bond with a third reactive group on a branched molecular chain attached to a surface of the electrode, and the biological enzyme forms a chemical bond with a second reactive group on a surface of the electrode.

3. The electrochemical biosensor of claim 2, wherein the transition metal complex is selected from at least one of ruthenium complexes, or osmium complexes, and the first reactive group is selected from at least one of an amino group, an aldehyde group, an epoxy group, or a carboxyl group.

4. The electrochemical biosensor of claim 2, wherein the second reactive group is selected from at least one of a hydroxyl group, an amino group, or a carboxyl group.

5. The electrochemical biosensor of claim 2, wherein the branched molecular chain comprises a polymer selected from at least one of polyepoxide, polyalkane, polyether, polyester, polyamide, polyamide ester, polyurethane, polysiloxane, or polycarbosilane, and the third reactive group is selected from at least one of a hydroxyl group, an amino group, an epoxy group, or a carboxyl group.

6. The electrochemical biosensor of claim 2, wherein the branched molecular chain has a relative molecular weight of 100 to 5,000.

7. The electrochemical biosensor of claim 1, wherein the biological enzyme is selected at least one of glucose oxidase, ethanol oxidase, lactate dehydrogenase, uric acid oxidase, acetylcholinesterase, or horseradish peroxidase.

8. The electrochemical biosensor of claim 1, further comprising a functional film layer disposed on the electrode.

9. A method for preparing an electrochemical biosensor, comprising:
step (a): activating an electrode comprising reactive groups, and chemically bonding a transition metal complex to the activated electrode to form a transition metal complex-modified electrode;
step (b): immobilizing a biological enzyme onto the transition metal complex-modified electrode to form the electrochemical biosensor.

10. The method of claim 9, wherein the transition metal complex-modified electrode in step (a) satisfies at least one of the following conditions:
(i) the reactive groups on the electrode include a second reactive group and/or a branched molecular chain comprising a third reactive group;
(ii) the activating an electrode comprising reactive groups comprises contacting the electrode with an activator solution selected at least one of: a mixture of dicyclohexylcarbodiimide and 4-dimethylaminopyridine; a mixture of diisopropylcarbodiimide and 4-dimethylaminopyridine; a mixture of dicyclohexylcarbodiimide and 4-pyrrolidinopyridine; a mixture of diisopropylcarbodiimide and 4-pyrrolidinopyridine; a mixture of 1-ethyl-3-(3-dimethylaminopropyl)carbodiimide and N-hydroxysuccinimide; an acyl chloride solution; or an anhydride solution; wherein the activation is performed at 0°C to 100°C for 0.5 hour to 72 hours;
(iii) in chemically bonding a transition metal complex to the activated electrode, placing the electrode in a transition metal complex solution for reaction under conditions comprising: reaction temperature: 0°C to 100°C; reaction duration: 1 hour to 72 hours; mass concentration of the transition metal complex solution: 0.01 wt% to 10 wt%; wherein the transition metal complex solution is selected from the group consisting of ruthenium complex solutions, osmium complex solutions, and combinations thereof.

11. The method of claim 9, wherein the biological enzyme is immobilized by:
(i) reacting the transition metal complex-modified electrode with a biological enzyme solution in the presence of an activator solution; or
(ii) contacting the transition metal complex-modified electrode with a biological enzyme solution and a crosslinking agent.

12. The method of claim 11, wherein:
the biological enzyme solution has a mass concentration of 0.01% to 10% and is selected at least one of glucose oxidase, ethanol oxidase, lactate dehydrogenase, uric acid oxidase, acetylcholinesterase, or horseradish peroxidase;
the crosslinking agent is selected at least one of glutaraldehyde, polyethylene glycol diglycidyl ether, dicarboxylic acid, dimethyl adiponimide, dimethyl adipic acid, dimethyl pyrimidate, dimethyl malonate, 3,3'-dithiobispropionimidate dimethyl ester dihydrochloride, 3,3' - dithiobis(sulfosuccinimidyl propionate), ethylene glycol bis(sulfosuccinimidyl succinate), carbodiimide hydrochloride, or N-cyclohexyl-N' -(2-morpholinoethyl)carbodiimide methyl p-toluenesulfonate.

13. Use of the electrochemical biosensor of any one of claims 1-8 for detecting glucose, lactic acid, ethanol, uric acid, or acetylcholine ester.
